# EUROPÄISCHE PATENTSCHRIFT

(11) **EP 0 316 633 B1**
(45) Veröffentlichungstag und Bekanntmachung des Hinweises auf die Patenterteilung: **27.01.1993**
(21) Anmeldenummer: 88117902.2
(22) Anmeldetag: 27.10.1988
(51) Int. Cl.: A61K 31/55, A61K 9/06

(54) **Azelastin enthaltende Arzneimittel zur Anwendung in der Nase und/oder am Auge**
Medicine containing azelastine for application in the nose and/or at the eye
Médicaments pour l'application dans le nez et/ou à l'oeil contenant de l'azélastine

(30) Priorität: 13.11.1987 DE 3738681
(43) Veröffentlichungstag der Anmeldung: 24.05.1989
(73) Patentinhaber: ASTA Medica Aktiengesellschaft, D-01277 Dresden (DE)
(72) Erfinder: Hettche, Helmut, Dr., D-6057 Dietzenbach (DE)
(74) Vertreter: Bezold, Gunter, Dr.

(56) Entgegenhaltungen:
- DE-A- 2 164 058
- DE-A- 3 530 793

## Beschreibung

Azelastin ist ein Phthalazinon-Derivat folgender Strukturformel:

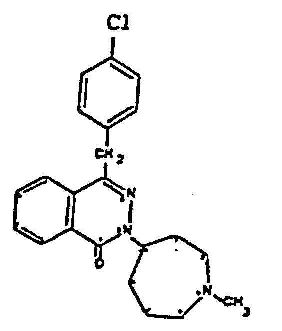

Die chemische Bezeichnung ist: 4-(4-Chlorbenzyl)-2-(perhydro-1-methyl-azepin-4-yl)-1-(2H)phthalazinon. Azelastin wird insbesondere zur Asthmaprophylaxe eingesetzt. Azelastin hat ebenfalls antiallergische und antihistaminische Eigenschaften, siehe deutsches Patent Nr. 21 64 058.

Es wurde nun gefunden, daß Azelastin und dessen physiologisch verträgliche Salze besonders vorteilhafte und überraschende Wirkungen aufweisen, wenn die Applikation entsprechender Zubereitungen in die Nase und/oder den Bindehautsack des Auges erfolgt.

So wird eine Beseitigung beziehungsweise deutliche Linderung nicht nur bei der allergisch bedingten Rhinitis sondern auch bei dem normalen banalen Schnupfen (beispielsweise durch Rhino-Viren verursacht) sowie dem vasomotorischen Schnupfen und den hierdurch ausgelösten Krankheitssymptomen erzielt. Überraschend ist hierbei, daß bei der lokalen nasalen Anwendung auch eine günstige Wirkung auf die Schleimhaut des Auges eintritt (Beseitigung beziehungsweise Linderung der Augenrötung und des Augenjuckens), sodaß sich häufig eine zusätzliche Anwendung von Augentropfen erübrigt.

Weitere Indikationen für die erfindungsgemäße Applikation/Anwendung sind beispielsweise: nicht spezifische Konjunctivitis, allergisch bedingte Konjunctivitis, allergisches Lidödem, katarrhliche Zustände im Auge oder der Nase, Coryza.

Bei der erfindungsgemäßen Anwendung wird außerdem überraschend die bei anderen Applikationen auftretende Müdigkeit nicht beobachtet.

Weiterhin besitzt Azelastin einen außerordentlich durchdringenden bitteren Geschmack, der bis jetzt jede orale Applikation von Azelastin-Lösungen verhindert hat, da solche Azelastin-Lösungen beziehungsweise Suspensionen von den Patienten abgelehnt werden.

Der Grad des Bittergeschmacks ist so intensiv, daß er sogar noch in einer Verdünnung von 1 : 10⁶ unangenehm wahrgenommen wird. Überraschend zeigte sich im Probandenversuch, daß beim Einsprühen der Azelastin-Zubereitungen gemäß der Erfindung in die Nase dieser bittere Geschmack nicht mehr in Erscheinung tritt, so daß es auf diese Weise möglich ist, Lösungen oder Suspensionen von Azelastin und dessen Salzen ohne Geschmacksbeeinträchtigung nasal zu applizieren. Auch beim Hinunterlaufen der eingesprühten Azelastin-Lösung beziehungsweise Suspension in den Rachenraum ist der bittere Geschmack kaum noch wahrnehmbar.

Die in dem deutschen Patent 2 164 058 erwähnten pharmazeutischen Zusammensetzungen sind hingegen für eine lokale Anwendung am Auge oder eine lokale Anwendung in der Nase nicht geeignet und führen beispielsweise bei einer derartigen Anwendung zu unangenehmen und schmerzhaften Reaktionen (Brennen, Beißen).

Im Zusammenhang mit pharmazeutischen Zubereitungen wird in diesem Patent auch lediglich auf orale und parenterale Zubereitungen hingewiesen.

Die deutsche Offenlegungsschrift 3 530 793 betrifft schließlich antiallergisch und astmaphrophylaktisch wirksame subsituierte Phenylphthalazinon-Derivate, die sich vom Azelastin durch Ersatz der sich am Homopiperazin befindlichen Methylgruppe durch den Benzyl-, Phenethyl-, Methoxyethyl- oder Allylrest unterscheiden, sowie Arzneimittel, die solche Verbindungen als Wirkstoffe enthalten. Die Appliktion dieser Arzneimittel kann unter anderem auch auf die Haut oder Schleimhaut, in Form von Lösungen, Aerosolen, Stäubepulver, beispielsweise auch nasal, erfolgen.

Aufgabe der Erfindung ist also die Bereitstellung eines gut verträglichen und verbesserten Mittels auf der Basis vo Azelastln beziehungsweise dessen Salzen zur nasalen Anwendung oder zur Anwendung am Auge und zwar zur Behandlung sowohl des allergisch bedingten wie auch des vasomotorischen und durch Rhino-Vien veursachten Schnupfens und dessen Begleiterscheinungen.

Die bevorzugte Ausführungsform der Erfindung stellt eine sterile und haltbare wässrige Lösung von Azelastin beiehungsweise dessen Salzen dar, die in Form von Tropfen, Salben, Cremes, Gelen, Einblaspulvern oder in einer ganz besonders bevorzugten Ausführung in Form eines Sprays (vorzugsweise Nasenspray) angewendet wird, wobei das Spray durch Verwendung einer üblichen Sprühquetschflasche oder eines Pumpenzerstäuers erzeugt werden kann. Weiterhin sind Druckgasaerosole möglich. Beispielsweise sollen pro Einzelsprühstoß 0,03 bis 3 mg Azelastin-Base feigesetzt werden.

Durch Anwendung von Nasentropfen oder eines Nasensprays ist die für die Behandlung des Schnupfens erforderliche Dosierung des Azelastins um etwa eine Zehnerpotenz niedriger und damit die Häufigkeit des Auftretens von Nebenwirkungen wesentlich geringer als bei der Anwendung von Azelastin in oral einzunehmenden Darreichungsformen wie Tabletten oder Säften, durch die der gesamte Körper mit der Wirkstubstanz überschwemmt wird. Insbesondere bei der Behandlung einer banalen Krankheit wie des Schnupfens ist eine niedrige Nebenwirkungsrate absolut geboten und erforderlich und stellt daher einen erheblichen medizinischen Fortschritt dar.

Als Lösungsmittel für die erfindungsgemäßen Zubereitungen kommen vorzugsweise in Frage: Wasser, gesättigte aliphatische ein- und mehrwertige Alkohole mit 2-3 C-Atomen, (zum Beispiel Ethanol, Isopropanol 1,2-Propylenglykol, Glycerin), flüssige Polyglykole (Mol-Gewicht 200 bis 600).

Vorzugsweise kommt als Lösungsmittel Wasser in Frage beziehungsweise Gemische von Wasser mit anderem physiologisch verträglichen Lösungsmitteln (beispielsweise den zuvorgenannten), wobei die Menge an letzteren in der wässrigen Mischung nicht über 15 Gew.% betragen soll.

Die Lösungen beziehungsweise Zubereitungen enthalten vorzugsweise Konservierungsmittel und Stabilisatoren. Als solche kommen zum Beispiel in Frage: Ethylendiamintetraessigsäure (Edetinsäure) und deren Alkalisalze (zum Beispiel Dialkalisalze wie Dinatriumsalz, Calciumsalz, Calcium-Natriumsalz), p-Hydroxybenzoesäure-Niederalkylester, Chlorhexidin (zum Beispiel in Form des Acetats oder Gluconats), Phenylquecksilberborat. Weiterhin kommen beispielsweise in Frage Natrium-(2-ethylmercurithio)-benzoat als "Thiomersal" allgemein bekannt, das in den erfindungsgemäßen Zubereitungen in einer Menge von 0,001 bis 0,05, vorzugsweise von 0,005 bis 0,02 zum Beispiel 0,01 % (Gewicht/Volumen bei flüsssigen Zubereitungen, sonst Gewicht/Gewicht) vorhanden sein kann. Weitere geeignete Konservierungsmittel sind: pharmazeutisch verwendbare quartäre Ammoniumverbindungen, zum Beispiel Cetylpyridiniumchlorid, Tetradecyltrimethylammoniumbromid, allgemein als "Cetrimid" bekannt, Benzyldimethyl-[2-[2-[p-(1,1,3,3-tetramethylbutyl)]-phenoxy]äthoxy]-ammoniumchlorid, allgemein als "Benzethoniumchlorid" bekannt, und Myristyly-pikoliniumchlorid, wobei jede dieser Verbindungen in einer Konzentration von 0,002 bis 0,05, zum Beispiel 0,02% (Gewicht/Volumen bei flüssigen Zubereitungen, sonst Gewicht/Gewicht) verwendet werden kann. Die bevorzugten Konservierungsmittel unter den quartären Ammoniumverbindungen sind jedoch die Alkylbenzyldimethylammoniumchloride und Mischungen von diesen zum Beispiel die allgemein als "Benzalkoniumchlorid" bekannten Verbindungen. Diese letztere besteht aus einer Mischung der Verbindungen der Formel, 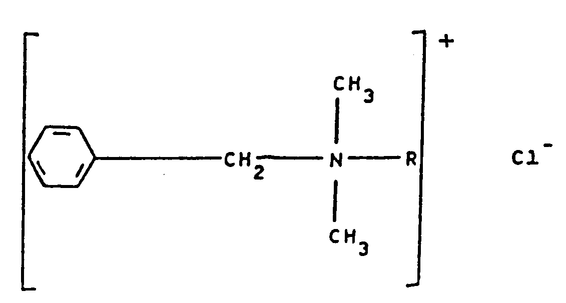 in der R eine Alkylgruppe mit der Formel CnH2n", wobei n eine ganze Zahl von 8 bis 18 bedeutet, darstellt. Besonders bevorzugt wird die Verwendung einer Mischung von Verbindungen, in denen n 10 bis 14 bedeutet, und insbesondere die spezielle Verbindung, in welcher R= C₁₂H₂₅ ist. "Benzalkoniumchlorid" und die Verbindungen der obigen Formel können in Konzentrationen von 0,005 bis 0,10, vorzugsweise von 0,005 bis 0,05, zum Beispiel von 0,01% (Gewicht/Volumen bei flüssigen Zubereitungen, sonst Gewicht/Gewicht) verwendet werden, und sie können gegebenenfalls in Kombination mit 0,2 bis 2,0, zum Beispiel 0,4% (Gewicht/Volumen) von 2-Phenyläthanol verwendet werden.

Die erfindungsgemäßen Zubereitungen (Lösungen, Suspensionen, auch ölige Lösungen beziehungsweise Suspensionen, Salben, Emulsionen, Cremes, Gele, Dosier-Aerosole) enthalten 0,0005 -2, vorzugsweise 0,001 bis 1 insbesondere 0,003 bis 0,5 % (Gewicht/Gewicht) Azelastin (bezogen auf die freie Azelastin Base). Liegt das Azelastin als Salz vor, sind diese Mengen entsprechend umzurechnen. Für die Augentropfen kommen dieselben Azelastin-Konzentrationen in Frage wie für die nasalen Formen.

Im Falle von Pulvern beträgt die Konzentration an Azelastin-Base 0,0005 bis 2 Gewichtsprozent bezogen auf die festen Trägerstoffe.

Bei Lösungen beträgt die Dosierung pro Nasenloch zum Beispiel 0,01 bis 0,2 ml, insbesondere 0,05 bis 0,15 ml, wobei eine solche Dosierung zum Beispiel 1 bis mehrmals, vorzugsweise 1 bis 5 mal täglich zu applizieren ist (gegebenenfalls auch stündlich).

Bei der Anwendung am Auge (Augentropfen) beträgt die Dosierung zum Beispiel 1 Tropfen (etwa 0,05 ml) der Lösung oder entsprechende Mengen der halbfesten Zubereitungsformen.

Als Säurekomponente für Salze des Azelastins kommen zum Beispiel in Frage: Halogenwasserstoffsäuren (HCI, HBr), Schwefelsäure, Phosphorsäuren (H₃P0₄, Metaphosphorsäure, Polyphosphorsäuren), Salpetersäure, organische Mono-, Di- oder Tricarbonsäuren von aliphatischen, alicyclischen, aromatischen oder heterocyclischen organischen Säuren (Embonsäure, Zitronensäure, Weinsäure) aliphatische und aromatische Sulfonsäuren (zum Beispiel Camphersulfonsäure).

Die Gesamtmenge an Konservierungsmittel in den Zubereitungen (Lösungen, Salben, usw.) beträgt pro 100 ml Lösung/Suspension beziehungsweise 100 g Zubereitung zwischen 0.001 bis 0,10, vorzugsweise 0.01 g.

Bei den Konservierungsmitteln kommen für Einzelstoffe zum Beispiel folgende Mengen in Frage:
Thiomersal 0,002 - 0,02 %
Benzalkoniumchlorid 0,002 bis 0.02 % (bei Kombination mit Thiomersal ist die Menge Thiomersal zu Beispiel = 0,002 bis 0,005 %;);
Chlorhexidinacetat beziehungsweise -gluconat 0,01 bis 0,02 %;
Phenylquecksilbernitrat, -borat, -acetat 0,002 - 0,004 %;
p-Hydroxybenzoesäureester (zum Beispiel Mischung des Methylesters und Propylesters 7 : 3): 0,05 - 0,15 vorzugsweise 0,1 %.

Vorzugsweise wird als Konservierungsmittel eine Kombination von Edetinsäure (zum Beispiel als Dinatriumsalz) und Benzalkoniumchlorid verwendet, wobei Edetinsäure in einer Konzentration von 0,05 bis 0,1 %, Benzalkoniumchlorid 0,005 bis 0,05 % vorzugsweise in einer Konzentration von 0,1 % eingesetzt wird.

Bei Lösungen/Suspensionen handelt es sich stets um Gewichtsprozent/Volumen, bei festen beziehungsweise halbfesten Zubereitungen um Gewichtsprozent/Gewicht der Zubereitung.

Als weitere Hilfsstoffe für die erfindungsgemäßen Zubereitungen kommen beispielsweise in Frage: Polyvinylpyrrolidon. Sorbitanfettsäureester wie Sorbitantrioleat, polyethoxylierte Sorbitanfettsäureester (zum Beispiel polyethoxyliertes Sorbitantrioleat), Sorbimacrogololeat, synthetische Amphotenside (Tritone@), Ethylenoxidether von octylphenolformaldehyd-Kondensationsprodukten, Phosphatide wie Lecithin, polyethoxylierte Fette, polyethoxylierte Oleotriglyceride, polyethoxylierte Fettalkohole. Polyethoxyliert bedeutet hierbei, daß die betreffenden Stoffe Polyoxyethylenketten enthalten, deren Polymerisationsgrad im allgemeinen zwischen 2 bis 40, insbesondere zwischen 10 bis 20 liegt. Diese Stoffe dienen vorzugsweise einer Löslichkeitsverbesserung der Azelastinkomponente.

Bei Zubereitungsformen, die Wasser enthalten, können gegebenenfalls zusätzlich Isotonisierungsmittel zugesetzt werden. Als Isotonisierungsmittel kommen zum Beispiel in Betracht: Saccharose, Glucose, Glycerin, Sorbit, 1,2-Propylenglykol, NaCI.

Die Isotonisierungsmittel bewirken die Einstellung der Zubereitungen auf den gleichen osmotischen Druck wie das Nasensekret. Für diesen Zweck ist von diesen Stoffen jeweils soviel zu verwenden, daß beispielsweise im Falle einer Lösung eine Gefrierpunktserniedrigung von 0,50 bis 0.56 ° C im Vergleich zu reinem Wasser erreicht wird. Bei Beispiel 1 wäre beispielsweise von solchen Stoffen eine solche Menge zu verwenden, die 68 g Natriumchlorid (0,68 %) isoosmotisch ist.

Im Beispiel 1 können statt NaCI pro 100 ml Lösung zum Beispiel verwendet werden:
Glucose 1 H₂0 3,81 g ; Saccharose 6,35 g ; Glycerin 2,2 g; 1,2-Propylenglykol 1,617 g ; Sorbit 3,84 g (Im Falle von Mischungen dieser Stoffe gegebenenfalls entsprechend weniger).

Den Lösungen können weiterhin Verdickungsmittel, die ein zu schnelles Abfließen der Lösung aus der Nase verhindern und der Lösung eine Viskosität von etwa 1,5 bis 3 vorzugsweise 2 mPa.s verleihen, zugesetzt werden. Als solche Verdickungsmittel kommen zum Beispiel in Frage: Cellulosederivate (zum Beispiel Celluloseether) bei denen die Cellulose-Hydroxygruppen teilweise mit niederen ungesättigen aliphatischen Alkoholen und/oder niederen ungesättigten aliphatischen Oxyalkoholen verethert sind (zum Beispiel Methylcellulose, Carboxymethylcellulose, Hydroxypropylmethylcellulose), Gelatine, Polyvinylpyrrolidon, Traganth, Ethoxose@ (wasserlösliches Binde- und Verdickungsmittel auf Basis von Ethylcellulose), Alginsäure, Polyvinylalkohol, Polyacrylsäure, Pektin und äquivalente Mittel. Falls diese Stoffe saure Gruppen enthalten kommen auch die entsprechenden physiologisch verträglichen Salze in Frage.

Im Falle der Verwendung von Hydroxy-propylcellulose werden beispielsweise 0,1 Gewichts% für diesen Zweck verwendet.

Den Zubereitungen können außerdem Puffersubstanzen wie Zitronensäure / Natriumhydrogenphosphat Borat-Puffer, Phosphate (Natriumdihydrogenorthophosphat, Dinatriumhydrogenphosphat), Tromethamol beziehungsweise äquivalente übliche Puffer zugesetzt werden, um beispielsweise einen pH-Wert der Zubereitung von 6 bis 7,5, vorzugsweise 6,5 bis 7,1 einzustellen.

Die Menge an Zitronensaure beträgt zum Beispiel 0,01 bis 0,14, vorzugsweise 0,04 bis 0,05 g, die Menge an Dinatriumhydrogenphosphat 0,1 bis 0,5, vorzugsweise 0,2 bis 0,3 g pro 100 ml Lösung. Die angegebenen Gewichtsmengen beziehen sich jeweils auf die wasserfreien Substanzen.

Bei den Lösungen und Suspensionen soll die maximale Gesamtkonzentration an Arzneimittel und Puffer weniger als 5 % insbesondere weniger als 2 % (Gewicht/Volumen) betragen.

Vorzugsweise wird für die nasale Applikation eine Losung oder Suspension verwendet, die als Aerosol, das heißt in Form einer feinen Verteilung in Luft oder einem anderen üblichen Trägergas zum Beispiel mittels eines üblichen Pumpzerstäubers appliziert wird.

Es ist jedoch auch eine Appplikation als Dosieraerosol möglich. Unter Dosieraerosolen sind Druckpakkungen zu verstehen, die das Azelastin beziehungsweise dessen Salze in Form einer Lösung oder Suspension in einem sogenannten Treibmittel enthalten. Als Treibmittel gelten unter Druck stehende flüssige, bei Normaldruck und Raumtemperatur gasförmige chlorierte fluorierte Kohlenwasserstoffe oder Mischungen von verschiedenen chlorierten fluorierten Kohlenwasserstoffen sowie Propan, Butan, Isobutan oder Mischungen dieser untereinander oder mit chlorierten, fluorierten Kohlenwasserstoffen. Die Druckpakkung weist ein Dosierventil auf, das bei Betätigung eine definierte Menge der Arzneistofflösung beziehungsweise -suspension freigibt. Durch die anschließend erfolgende schlagartige Verdampfung des Treibmittels wird die Lösung beziehungsweise Suspension von Azelastin in feinste Tröpfchen beziehungsweise Partikelchen zerissen, die in die Nase gesprüht werden oder für eine Einatmung in die Nase zur Verfügung stehen. Man bedient sich zur Betätigung des Ventils und zur Verbringung der versprühten Suspension in die Nase bestimmter Applikatoren aus Kunststoff. Als Treibmittel kommen aber auch in Frage: C0₂ Distickstoffoxid, Preßluft.

Bei der Applikation als Aerosol kann auch ein üblicher Adapter verwendet werden.

Bei Verwendung von Suspensionen soll die maximale Teilchengröße der festen Stoffe (Azelastin + Hilfstoffe) nicht größer als 30 um sein.

Bei der Anwendung in Form eines Einblaspulvers soll die maximale Teilchengröße der Stoffe nicht größer als 20 um sein.

Es handelt sich hierbei beispielsweise um ein Verstäuben von festem Azelastin oder dessen Salzen. In diesem Falle wird beispielsweise Azelastin beziehungsweise sein Salz mit inerten Trägerstoffen vermischt beziehungsweise auf inerte Trägerstoffe aufgezogen. Als Trägerstoffe kommen zum Beispiel infrage: Zucker wie Glucose, Saccharose, Lactose, Fructose. Sodann Stärke oder Stärkederivate, Oligosaccharide wie Dextrine, Cyclodextrine und deren Derivate, Polyvinylpyrrolidon, Alginsäure, Tylose, Kieselsäure, Cellulose, Cellulosederivate (zum Beispiel Celluloseether), Zuckeralkohole wie Mannit oder Sorbit, Calciumcarbonat, Calciumphosphat. Die Konzentration von Azelastin beträgt 1 Gewichtsteil Azelastin auf 50 bis 200 000 Gewichtsteile Trägersubstanz (0,0005 bis 2 % Azelastin).

### Beispiel 1

### Nasenspray oder Nasentropfen oder Augentropfen mit 0,1 % Azelastinhydrochlorid als Wirkstoff

In 9,00 kg Wasser werden in folgender Reihenfolge gelöst:
10 g Azelastinhydrochlorid, 5 g Edetinsäure-Dinatriumsalz.2 H₂0, 68 g Natriumchlorid, 1,25 g Alkylbenzyldimethylammoniumchlorid (Benzalkoniumchlorid), 4,38 g Citronensäure, 64,8 g Natriummonohydrogenphosphat.12 H₂0 sowie 10 g Hydroxypropyl-Methylcellulose^{l.}) Die erhaltene Lösung wird mit Wasser auf 10,05 kg = 10 Liter aufgefüllt und nach sorgfältigem Mischen über ein Membranfilter der Porenweite 0,2 um filtriert, wobei 500 ml Vorlauf verworfen werden. Das Filtrat hat einen pH-Wert von 6,8 ± 0,3. Die Abfüllung erfolgt in Kunststoff-Flaschen, die mit einem üblichen Sprüheinsatz oder in Kunststoff- beziehungsweise
   ') Handelsprodukt zum Beispiel Methocel@ E4M premium.

   Glasflaschen, die mit einem üblichen Pumpensprüher verschlossen werden. Im letzteren Fall werden zum Beispiel Pumpen mit Nasensprühaufsatz verwendet, die pro Betätigung circa 0,14 ml Lösung versprühen. Damit werden pro Betätigung 0,14 mg Azelastinhydrochlorid in Form der Lösung in die Nase gesprüht.

Füllt man das oben erhaltene Filtrat in für Nasentropfen oder Augentropfen übliche Flaschen mit Tropfpipette ab, so kann die Lösung mittels Tropfpipette in die Nase oder ins Auge geträufelt werden.

### Beispiel 2:

### Nasensalbe mit 0,1 % Azelastinhydrochlorid

In einem heizbaren Behälter werden 5 kg Polyoxyethylenstearat^{*}, 8 kg Cetylstearylalkohol (Lanette@0), 20 kg weißes Vaselin, 15 kg flüssiges Paraffin und 0,5 kg Siliconöl zusammen geschmolzen. In die Schmelze (Temperatur der Schmelze 80 ° C) werden 126 g p-Hydroxybenzoesäuremethylester und 53 g pHydroxybenzoesäurepropylester gelöst. Anschließend wird eine auf 70 ° C erwärmte Lösung von 0,1 kg Azelastinhydrochlorid, 140 g p-Hydroxybenzoesäuremethylester und 60 g p-Hydroxybenzoesäurepropylester in 51,021 kg gereinigtem Wasser mit Hilfe eines hochtourigen Rührers einemulgiert und die erhaltene Emulsion bis zum Erkalten gerührt und in regelmäßigen Zeitabständen wiederholt homogenisiert.

Die Abfüllung der Salbe erfolgt in Tuben, die vor dem Gewinde eine röhrenförmige Verlängerung aufweisen und daher zur Applikation der Salbe in die Nase besonders geeignet sind.

### Beispiel 3:

### Dosieraerosol mit einer Abgabe von 0,5 mg Azelastinhydrochlorid pro Hub

In einem geeigneten Kühlbehälter werden circa 8,0 kg eines Gemisches aus 70 Gewichtsteilen Difluordichlormethan und 30 Gewichtsteilen 1,2-Dichlortetrafluorethan auf etwa -55 ° C abgekühlt. In diesem Gemisch wird bei - 55°C eine Mischung aus 0,086 kg vorgekühltem Sorbitantrioleat und 0,8600 kg vorgekühltem Trichlorfluormethan unter Rühren gelöst. In die so erhaltene Lösung werden dann unter intensivem Rühren 0,0688 kg mikronisiertes Azelastinhydrochlorid und 0,0688 kg mikronisierte Lactose portionsweise eingetragen. Durch Zugabe von weiterem auf etwa -55°C gekühlten Gemisch aus 70 Gewichtsteilen Difluordichlormethan und 30 Gewichtsteilen 1,2-Dichlortetrafluorethan wird das Gesamtgewicht der erhaltenen Suspension auf 9,547 kg gebracht.
Nach dem Verschließen des Kühlbehälters wird die Suspension unter intensivem Rühren erneut auf etwa -55°C abgekühlt. Sie ist danach abfüllfertig.

Unter fortgesetztem Rühren wird die Suspension in übliche geeignete Aluminium-Monobloc-Dosen abgefüllt. Die Monobloc-Dosen werden unmittelbar nach Einfüllung der Suspension mit hierfür üblichen Dosierventilen verschlossen, die pro Ventilbetätigung 0,05 ml Suspension freisetzen. Bei der Betätigung des Ventils werden damit 0,5 mg Azelastinhydrochlorid abgegeben. Die Abgabe erfolgt in Verbindung mit einem üblichen Applikator, der die Einbringung der Wirkungssubstanz in die Nase des Patienten erlaubt.

### Beispiel 4:

### Augentropfen mit 0,05 % Azelastinhydrochlorid.

140 g Polyvinylalkohol (Handelsname zum Beispiel: Mowiol@ 26 - 88 / Hoechst AG, Frankfurt 80) werden in 4 Liter kaltes Wasser für Injektionszwecke eingerührt, die Suspension auf 90 ° C erwärmt und 45 Minuten bei dieser Temperatur belassen. Die erhaltene Lösung wird nach dem Abkühlen mit folgenden Lösungen gemischt: 5 g Azelastinhydrochlorid in 1 Liter Wasser für Injektionszwecke, 0,2 g Phenylquecksilbernitrat in 2 Liter Wasser für Injektionszwecke, 70 g Natriumchlorid in 1 Liter Wasser für Injektionszwecke.

Die Mischung wird durch Zusatz von 0,1 N Natronlauge auf einen pH-Wert von 6,8 eingestellt, mit einer Lösung von 15 g Natriumdihydrogenphosphat.2 H₂0 und 21 g Dinatriumhydrogenphosphat.2 H₂0 in 1 Liter Wasser für Injektionszwecke vermischt und mit Wasser für Injektionszwecke auf 10 Liter aufgefüllt.

Nach sorgfältigem Mischen wird die Lösung durch ein Membranfilter der Porenweite 0,2 um mit Glasfaservorfilter filtriert und nach Verwerfen eines Vorlaufs von 500 ml unter aseptischen Bedingungen in sterile Augentropfenflaschen abgefüllt.
^{*} Polyoxyethylen-40-stearat, feste, weiße bis cremefarbene Masse, D.²⁵ ca 1,1, F. 40 - 44°C, Erstarrungspunkt ca. 41°C.

## Patentansprüche

1. Arzneimittel zur nasalen Anwendung oder zur Anwendung am Auge, welches 0,0005 bis 2 % (Gewicht/Gewicht) Azelastin enthält, wobei das Azelastin auch in Form eines physiologisch verträglichen Salzes vorliegen kann.

2. Verwendung von Azelastin oder dessen physiologisch verträglichen Salzen zur Herstellung eines Arzneimittels zur nasalen oder okularen Behandlung von allergisch bedingtem oder vasomotorischem oder durch Rhino-Viren verursachten Schnupfen beziehungsweise Krankheitssymptomen.

3. Arzneimittel nach Anspruch 1,
dadurch gekennzeichnet,
daß es ein pharmazeutisches verwendbares Konservierungsmittel in einer Menge von 0,001 bis 0,1 % (bei Lösungen - Gewicht pro Volumen der Lösung; bei festen Zubereitungen Gewicht pro Gewicht der Zubereitung) enthält.

4. Arzneimittel nach Anspruch 1
dadurch gekennzeichnet, daß es eine wässrige Lösung darstellt.

5. Lösung nach Anspruch 1,
dadurch gekennzeichnet,
daß sie 0,001 bis 0,05 % (Gewicht/Volumen Lösung) Natrium-2-(ethylmercurithio)-benzoat oder 0,001 bis 0,1 % (Gewicht/Volumen Lösung) Alkylbenzyldimethylammoniumchlorid enthält.

6. Verfahren zur Herstellung eines Arzneimittels zur nasalen Anwendung oder zur Anwendung am Auge, welches 0,0005 bis 2 % (Gewicht/Gewicht) Azelastin enthält, wobei das Azelastin auch in Form eines physiologisch verträglichen Salzes vorliegen kann.

7. Verfahren zur Herstellung von sterilen Azelastinhaltigen Zubereitungen zur Anwendung in der Nase und/oder am Auge
dadurch gekennzeichnet,
daß man bei Temperaturen zwischen -55 und + 80 ° C
a) 1 bis 1000 mg Azelastin oder ein physiologisch verträgliches Satz des Azelastins in 50 bis 200 ml Wasser, welches gegebenenfalls bis zu 15 Gewichts% weitere mit Wasser mischbare verträgliche Lösungsmittel enthalten kann, unter gleichzeitigem oder nachfolgendem Zusatz von
1 bis 400 mg Konservierungsstoffen
50 bis 4000 mg Stabilisierungsmitteln beziehungsweise löslichkeitsverbessernden Stoffen auflöst,
und gegebenenfalls die Lösung mittels Puffer auf einen pH-Wert von 6.5 bis 7.1 einstellt sowie gegebenenfalls Isotonisierungsmittel zusetzt; oder
b) die in a) erhaltene Lösung durch Zusatz von 0,5 bis 10 g Verdickungsmittel in ein Gel überführt; oder
c) 7,5 mg bis 10 g Azelastin oder ein physiologisch verträgliches Satz des Azelastins in 400 bis 900 ml Wasser unter gleichzeitigem oder nachfolgendem Zusatz von 10 - 200 mg Konservierungsstoffen auflöst, die Lösung in 100 -600 g einer Schmelze aus Kohlenwasserstoffgemischen und/oder Silikonen und/oder anderen fettartigen Bestandteilen (Fetten, Fettalkoholen) sowie Emulgatoren einemulgiert und die erhaltene Emulsion homogenisiert und dabei abkühlt bis auf Raumtemperatur; oder
d) 0,05 bis 100 g Azelastin oder ein physiologisch verträgliches Salz des Azelastins in 5 bis 10 kg eines Gemisches aus chlorierten fluorierten Kohlenwasserstoffen und/oder Kohlenwasserstoffen unter Zusatz von 25 bis 150 g Sorbitantrioleat dispergiert und die erhaltene Suspension in Dosen abfüllt, die mit Dosierventilen verschlossen sind beziehungsweise werden, welche pro Betätigung 0,025 bis 0,1 ml der Suspension freisetzen; oder
e) 5 mg bis 10 g Azelastin oder ein physiologisch verträgliches Salz des Azelastins mit 500 bis 1000 g eines physiologisch inerten Trägerstoffes mischt beziehungsweise die Lösung der genannten Menge Azelastin oder eines physiologisch verträglichen Salzes von Azelastin gegebenenfalls portionsweise mit der genannten Menge inertem Trägerstoff mischt und nachfolgend das Lösungsmittel wieder abdampft und die erhaltene Mischung in einer Menge von 20 bis 1000 mg in Hartgelatinekapseln oder Tütchen abfüllt.

## Claims

1. Pharmaceutical preparation for nasal application or for use on the eye, containing from 0.0005 to 2% (weight/weight) of azelastine which may be present in the form of a physiologically acceptable salt.

2. The use of azelastine or its physiologically acceptable salts for the preparation of pharmaceutical preparations for nasal or ocular treatment of nasal catarrh or disease symptoms of allergic or vasomotor or rhino-viral origin.

3. Pharmaceutical preparation according to Claim 1, characterised in that it contains a pharmaceutically acceptable preservative in a quantity of from 0.001 to 0.1% (in solutions - weight per volume of the solution; in solid preparations - weight per weight of the preparation).

4. Pharmaceutical preparation according to Claim 1, characterised in that it is an aqueous solution.

5. Solution according to Claim 1, characterised in that it contains from 0.001 to 0.05% (weight/volume of solution) of sodium-2-(ethylmercurithio)-benzoate or from 0.001 to 0.1% (weight/volume of solution) of alkyl benzyl dimethylammonium chloride.

6. A process for the preparation of a pharmaceutical preparation for nasal application or for application to the eye, containing from 0.0005 to 2 % (weight/weight) of azelastine which may also be in the form of a physiologically acceptable salt.

7. A process for the preparation of sterile preparations containing azelastine for use in the nose and/or on the eye, characterised in that, at temperatures of from -55 ° C to + 80 ° C,
a) from 1 to 1000 mg of azelastine or a physiologically acceptable salt of azelastine is dissolved in 50 to 200 ml of water optionally containing up to 15% by weight of other water-miscible acceptable solvents, with simultaneous or subsequent addition of
from 1 to 400 mg of preservatives and
from 50 to 4000 mg of stabilizing agents or solubility improving substances,
and the solution is optionally adjusted to a pH of from 6.5 to 7.1 by means of buffer and an agent to render it isotonic is optionally added; or
b) the solution obtained in a) is converted into a gel by the addition of from 0.5 to 10 g of thickener or
c) from 7.5 mg to 10 g of azelastine or a physiologically acceptable salt of azelastine are dissolved in 400 to 900 ml of water with simultaneous or subsequent addition of from 10 - 200 mg of preservatives, the solution is emulsified in from 100 - 600 g of a melt of hydrocarbon mixtures and/or silicones and/or other fat-like components (fats, fatty alcohols) and emulsifiers and the emulsion obtained is homogenised and cooled to room temperature; or
d) from 0.05 to 100 g of azelastine or a physiologically acceptable salt of azelastine are dispersed in 5 to 10 kg of a mixture of chlorinated fluorinated hydrocarbons and/or hydrocarbons with the addition of from 25 to 150 g of sorbitan trioleate and the resulting suspension is filled into cans closed with dosing valves which release from 0.025 to 0.1 ml of the suspension every time they are operated; or
e) from 5 mg to 10 g of azelastine or a physiologically acceptable salt of azelastine are mixed with from 500 to 1000 g of a physiologically inert carrier or the solution of the above mentioned quantity of azelastine or of a physiologically acceptable salt of azelastine is optionally mixed portionwise with the said quantity of inert carrier and the solvent is subsequently evaporated off and the mixture obtained is introduced into hard gelatine capsules or sachets in a quantity of from 20 to 1000 mg.

## Revendications

1. Médicament à usage natal ou à usage ophtalmique, qui contient de 0,0005 à 2% (poids/poids) d'azélastine, l'azélastine pouvant aussi se présenter sous forme d'un sel acceptable physiologiquement.

2. Utilisation d'azélastine ou de sels physiologiquement acceptables de celle-ci pour la préparation d'un médicament pour le traitement nasal ou oculaire de coryzas ou de symptômes pathologiques d'origine allergique, vasomotrice ou rhinovirale.

3. Médicament selon la revendication 1, caractérisé en ce qu'il contient un agent de conservation utilisable en pharmacie, à raison de 0,001 à 0,1% (en cas de solutions - poids par rapport au volume de la solution; en cas de préparations solides - poids par rapport au poids de la préparation).

4. Médicament selon la revendication 1, caractérisé en ce qu'il s'agit d'une solution aqueuse.

5. Solution selon la revendication 1, caractérisée en ce qu'elle contient de 0,001 à 0,05% (poids/volume de solution) de 2-(éthylmercurithio)-benzoate de sodium ou de 0,001 à 0,1% (poids/volume de solution) de chlorure d'alkylbenzyldiméthylammonium.

6. Procédé de préparation d'un médicament à usage nasal ou à usage ophtalmique, qui contient de 0,0005 à 2% (poids/poids) d'azélastine, l'azélastine pouvant aussi se présenter sous forme d'un sel acceptable physiologiquement.

7. Procédé de préparation de compositions stériles contenant de l'azélastine à usage nasal et/ou ophtalmique, caractérisé en ce que
a) on dissout, à des températures comprises entre -55 et + 80 ° C, 1 à 1000 mg d'azélastine ou d'un sel physiologiquement acceptable de l'azélastine dans 50 à 200 ml d'eau qui peut éventuellement contenir jusqu'à 15% en poids d'autres solvants acceptables et miscibles à l'eau, avec addition simultanée ou ultérieure de
1 à 400 mg d'agents de conservation,
50 à 4000 mg de stabilisants ou de substances améliorant la solubilité,
on règle éventuellement la solution à un pH de 6,5 à 7,1 au moyen d'un tampon et on ajoute éventuellement des agents d'isotonisation; ou
b) on transforme en un gel la solution obtenue en a), par addition de 0,5 à 10 g d'épaississant; ou
c) on dissout 7,5 mg à 10 g d'azélastine ou d'un sel physiologiquement acceptable de l'azélastine dans 400 à 900 ml d'eau avec addition simultanée ou ultérieure de 10 à 200 mg d'agents de conservation, on émulsionne la solution dans 100-600 g d'une masse fondue composée d'un mélange d'hydrocarbures et/ou de silicones et/ou d'autres constituants lipoïdiques (graisses, alcools gras) ainsi que d'émulsifiants, on homogénéise l'émulsion obtenue et on la refroidit jusqu'à la température ambiante; ou
d) on disperse 0,05 à 100 g d'azélastine ou d'un sel physiologiquement acceptable de l'azélastine dans 5 à 10 kg d'un mélange d'hydrocarbures chlorofluorés et/ou d'hydrocarbures, avec addition de 25 à 150 g de trioléate de sorbitanne, et on remplit, avec la suspension obtenue, des récipients qui sont fermés ou que l'on ferme avec des valves de dosage qui libèrent de 0,025 à 0,1 ml de la suspension à chaque manoeuvre; ou
e) on mélange de 5 mg à 10 g d'azélastine ou d'un sel physiologiquement acceptable de l'azélastine avec 500 à 1000 g d'un excipient physiologiquement inerte, ou on mélange la solution de ladite quantité d'azélastine ou d'un sel physiologiquement acceptable de l'azélastine, éventuellement par portions, avec ladite quantité d'excipient inerte, après quoi on évapore le solvant et on remplit, avec le mélange obtenu, des capsules de gélatine dure ou des sachets, à raison de 20 à 1000 mg.
